# DEMANDE DE BREVET EUROPEEN

(11) **EP 2 233 125 A2**
(43) Date de publication de la demande: **29.09.2010**
(21) Numéro de dépôt: 10153239.8
(22) Date de dépôt: 07.11.2005
(51) Int. Cl.: A61K 8/06, A61K 8/88, A61Q 1/00, A61Q 19/00

(54) **Composition cosmétique comprenant une poudre fine et poreuse**

(30) Priorité: 30.11.2004 FR 0412690
(62) Demande divisionnaire de: 05024240.3
(71) Demandeur: Arkema France, 92700 Colombes (FR)
(72) Inventeur: Loyen, Karine, 27500, Pont Audemer (FR); Kohler, Sophie, 78420, Carrieres-sur-Seine (FR)

(57) **Abrégé**

Notre invention est une émulsion eau/huile comprenant une poudre poreuse, les particules de la poudre ayant les caractéristiques suivantes :
- une surface spécifique apparente de 1 à 299 m²/g mesurée par absorption d'azote selon la méthode BET ISO 9277;
- une Absorption d'huile de lin entre 45g/100g de poudre et 160g/100g de poudre mesurée selon ISO 787-5.

Le diamètre moyen des particules de la poudre est compris entre 2 µm à 100 µm, de préférence de 2 à 50 µm, encore plus avantageusement de 2 à 20 µm. La poudre est choisie parmi une poudre de polyamides, de polyesteramides, de polyuréthanes, de poly(méthyle)méthacrylates, de polymères acryliques, de polyesters, de silicones, de polyéthylènes et de silices.

Notre invention a également pour objet l'utilisation de l'émulsion pour obtenir un maquillage et/ou de soin de la peau non brillant, non huileux, non poisseux et/ou non collant ainsi que l'utilisation de la poudre fine pour améliorer le toucher et/ou l'aspect d'une émulsion cosmétique.

## Description

La présente invention est relative à des compositions cosmétiques utilisées dans le domaine du soin et du maquillage. Ce sont des émulsions comprenant principalement une phase aqueuse et une phase grasse et comprenant en plus une poudre fine et poreuse. L'invention concerne plus particulièrement les compositions cosmétiques pour soin et/ou maquillage présentant une phase grasse continue, cette dernière pouvant être composée de différents types d'huiles, volatiles ou non volatiles, d'origine minérale, animale, végétale ou de synthèse.

L'utilisation dans ces compositions cosmétiques selon l'invention de tensioactifs, d'épaississants, et plus généralement d'agents de surface permet d'obtenir une dispersion stable d'une phase dans l'autre. On peut également avoir dans ces compositions des additifs tels que des conservateurs et des parfums mais aussi des actifs cosmétiques tels que des hydratants (polyols), des anti-UV, des antirides, des autobronzants, des filmogènes, des antioxydants et bien d'autres.

L'invention a également pour objet un procédé de maquillage et/ou de soin des matières kératiniques comme la peau, les lèvres, les ongles, les cheveux, les cils, les sourcils, les poils d'être humain, comprenant l'application de la composition selon l'invention sur les matières kératiniques.

La composition selon l'invention peut être une composition de maquillage et/ou de soin des matières kératiniques, en particulier une composition de soin du visage (crème ou fluide), une composition de soin du corps (hydratant, amincissant), une composition de crème solaire résistante à l'eau (« waterproof » en terminologie anglo-saxonne) ou non résistante à l'eau, une composition de maquillage de la peau, telle qu'un fond de teint, un fard à paupières, un fard à joue, un produit anti-cernes, un produit de maquillage du corps. Les compositions de maquillage comprennent en générale, d'une part, une phase pulvérulente comportant notamment des pigments et des charges et d'autre part, une phase grasse, les charges et la phase grasse étant destinés à conférer au produit fini une certaine densité, à donner une douceur et une propriété émolliente au produit de maquillage et à favoriser son adhérence sur la peau.

Les compositions de type phase grasse continue présentent de nombreux avantages sur le plan cosmétique et galénique. La phase grasse selon l'invention comprend des corps gras solides ou liquides, d'origine végétale, minérale, animale ou de synthèse. On peut citer par exemple les esters, les alcools gras, les acides gras, les hydrocarbures comprenant essentiellement des atomes de carbone et d'hydrogène et éventuellement des atomes d'azote, d'oxygène. Ces formulations sont en effet avantageusement compatibles avec la peau et ses constituants lipidiques. La formation d'un film à la surface de la peau contribue à limiter l'évaporation de l'eau contenue dans les couches de la peau et à préserver l'hydratation de celle-ci assurant ainsi une bonne protection de la peau vis-à-vis du dessèchement. On peut aussi citer les huiles siliconées et les huiles fluorées.

De plus, les actifs (hydratant, filtres UV chimiques ou physiques) étant le plus souvent lipophiles, ils pourront être dispersés ou dissous plus facilement dans la phase grasse continue qui les véhiculera au niveau des couches constitutives de la peau, assurant une bonne distribution de ces actifs. La phase grasse continue et hydrophobe constitue également un milieu protecteur pour ces actifs. En effet, les fluides corporels (larmes, transpiration) ou l'eau ont tendance à éliminer ces actifs de la surface de la peau par lavage ou ruissellement. L'incompatibilité de l'eau avec la phase grasse continue empêche ou limite fortement cette élimination. Ceci est particulièrement appréciable pour les crèmes solaires protectrices de la peau de type « waterproof » qui doivent conserver leur efficacité de protection vis-à-vis des rayonnements UV même après un bain.

Cependant, les compositions cosmétiques à phase grasse continue provoquent très souvent des désagréments à l'application qui limitent parfois leur utilisation par les consommateurs. En effet, le film gras continu à la surface de la peau provoque une sensation de collant, d'huileux et de poisseux, qui n'est plus acceptée par les consommateurs d'aujourd'hui. Par ailleurs, l'aspect brillant et huileux nuit aux propriétés cosmétiques et esthétiques de ces crèmes. L'application d'un maquillage après l'application de crème de jour ou solaire est rendue difficile à cause de cet effet collant qui interfère dans l'étalement du maquillage sur la peau. L'application du maquillage est donc perturbée, provoquant des inhomogénéités. De plus, au cours du temps, on assiste à une mauvaise tenue du maquillage ainsi qu'à un transfert et une perte des couleurs de ce dernier.

Pour réduire l'aspect huileux, collant et poisseux de ces compositions à phase grasse continue, il est connu d'ajouter des huiles volatiles telles que les huiles de silicone mais elles ne jouent pas le rôle protecteur associé à une phase grasse continue et n'ont pas de propriétés humectantes de la peau. Par ailleurs, les polyols ajoutés généralement dans les formulations pour leurs propriétés humectantes et hydratantes de la peau apportent un effet poisseux persistant, indésirable sur la peau, qui s'ajoute à celui des huiles cosmétiques de la composition.

Il est donc important de fabriquer une composition cosmétique répondant à la fois au problème sensoriel et esthétique tout en remplissant son rôle protecteur vis-à-vis de la peau tel qu'évoqué plus haut. Une émulsion eau/huile comprenant une poudre fine et poreuse selon l'invention permet de résoudre ce problème technique. Elle réduit notamment de façon significative l'effet gras et poisseux apporté par les huiles et/ou les polyols des compositions cosmétiques.

Par ailleurs, d'après le document EP 1582194 de UBE INDUSTRIES, on connaît une composition cosmétique comprenant une base cosmétique, un parfum et une poudre formée de particules de polyamide (abrégé de PA) sphériques, cylindriques ou en forme d'haltères. Ces particules sont poreuses, ont un diamètre moyen de 1 à 30 µm, une surface spécifique de 5 m²/g ou plus, une absorption d'huile de lin de 200 ml/100g ou plus, une cristallinité de 40% ou plus et un ratio du diamètre moyen en volume par rapport au diamètre moyen en nombre de 1,0 à 1,5. Les compositions cosmétiques décrites dans ce document contiennent préférablement de 3 à 10% en poids de phase grasse par rapport au poids total de la composition. La poudre de ces compositions cosmétiques provoque un effet de diffusion de la lumière à la surface de la peau et a une forte capacité d'absorption du sébum, ce qui évite l'apparition de zones grasses et brillantes sur le visage.

Le document FR 2854064 de L'OREAL est relatif à une composition de maquillage pouvant absorber le sébum permettant de disposer ainsi d'un maquillage présentant de bonnes propriétés cosmétiques au cours du temps en particulier présentant une bonne tenue aux frottements, des propriétés non transfert, une bonne homogénéité, un maintien de la couleur initiale du maquillage et une absence de brillance (tenue de la matité). Cette composition comprend :
- une première poudre absorbant le sébum ayant une surface spécifique BET (voir ci-dessous) supérieure ou égale à 300 m2/g de préférence supérieure à 500 m2/g, associée avec
- une seconde poudre ayant une énergie de surface critique particulière qui ne s'imprègne pas du sébum et empêchant ce dernier de modifier les propriétés cosmétiques du maquillage.

L'invention a pour objet une émulsion eau/huile comprenant une poudre poreuse, les particules de la poudre ayant les caractéristiques suivantes :
- une surface spécifique apparente de 1 à 299 m²/g mesurée par absorption d'azote selon la méthode BET ISO 9277;
- une Absorption d'huile de lin entre 45g/100g de poudre et 160g/100g de poudre mesurée selon ISO 787-5.

Selon un mode de réalisation, l'émulsion est **caractérisée en ce que** la poudre présente une absorption d'huile de lin est de 50 g/100g de poudre à 150 g/100g de poudre.

Selon un mode de réalisation, l'émulsion est **caractérisée en ce que** la surface spécifique apparente est de 0,5 à 150 m²/g.

Selon un mode de réalisation, l'émulsion est **caractérisée en ce que** la surface spécifique apparente est de 0,5 à 100 m²/g, avantageusement de 0,5 à 50 m²/g, encore plus avantageusement de 0,5 à 40 m²/g.

Selon un mode de réalisation, l'émulsion est **caractérisée en ce que** les particules de poudre ont un diamètre moyen allant de 2 µm à 100 µm, de préférence de 2 à 50 µm, encore plus avantageusement de 2 à 20 µm.

Selon un mode de réalisation, l'émulsion est **caractérisée en ce que** les particules de poudre ont une forme sphéroïdale.

Selon un mode de réalisation, l'émulsion est **caractérisée en ce que** la poudre est choisie parmi une poudre de polyamide, de polyesteramides, de polyuréthanes, de poly(méthyle)méthacrylates, de polymères acryliques, de polyesters, de silicones, de polyéthylènes et de silices.

Selon un mode de réalisation, l'émulsion est **caractérisée en ce que** sa composition est la suivante :
- 10 à 75%, de préférence 30 à 65% d'une phase aqueuse ;
- 0,1 à 30%, de référence 1 à 20% de poudre poreuse; et
- 89,9 à 24,9% d'une phase grasse, le total faisant 100%.

Selon un mode de réalisation, l'émulsion est **caractérisée en ce que** la phase grasse comprend moins de 25% (en poids par rapport à la composition totale) d'huile volatile.

Selon un mode de réalisation, l'émulsion est **caractérisée en ce que** l'huile volatile est une huile siliconée.

Selon un mode de réalisation, l'émulsion est **caractérisée en ce que** la phase aqueuse comprend de 10 à 60% de polyols.

Selon un mode de réalisation, l'émulsion est caractérisée par le fait qu'elle comprend un ingrédient cosmétique choisi parmi les antioxydants, les parfums, les conservateurs, les neutralisants, les tensioactifs, les polymères filmogènes, les épaississants, les bloqueurs de rayonnements ultra violet, les vitamines, les matières colorantes, les stabilisants d'émulsion, les hydratants, les composés auto-bronzants, les actifs antirides et leurs mélanges.

Selon un mode de réalisation, l'émulsion est **caractérisée en ce que** c'est une crème ou un fluide de soin du visage, une crème ou un fluide de soin du corps hydratant et/ou amincissant, une crème solaire résistante à l'eau ou non résistante à l'eau, un fond de teint, un fard à paupières, un fard à joue, un produit anti-cernes ou un produit de maquillage du corps.

L'invention a également trait à l'utilisation d'une émulsion pour fabriquer un maquillage et/ou de soin de la peau non brillant, non huileux, non poisseux et/ou non collant.

L'invention concerne aussi un procédé cosmétique de maquillage et/ou de soin des matières kératiniques, comprenant l'application sur ces matières d'une émulsion telle que décrite précédemment.

L'invention a pour objet l'utilisation d'une poudre fine et poreuse présentant les caractéristiques suivantes:
- une surface spécifique apparente de 1 à 299 m²/g mesurée par absorption d'azote selon la méthode BET ISO 9277;
- une Absorption d'huile de lin entre 45g/100g de poudre et 160g/100g de poudre mesurée selon ISO 787-5 ;
pour améliorer le toucher et/ou l'aspect d'une émulsion cosmétique eau/huile.

Selon un mode de réalisation, l'utilisation de la poudre fine et poreuse est **caractérisée en ce que** la surface spécifique apparente de ladite poudre est de 0,5 à 150 m²/g mesurée par absorption d'azote selon la méthode BET ISO 9277.

Selon un mode de réalisation, l'utilisation de la poudre fine et poreuse est **caractérisée en ce que** les particules de poudre ont un diamètre moyen allant de 5 à 20 µm.

Selon un mode de réalisation, l'utilisation de la poudre fine et poreuse est **caractérisée en ce que** les particules de poudre ont une forme sphéroïdale.

L'invention va maintenant être décrite plus en détails.

### POUDRE

S'agissant de la poudre de polyamides homopolymères ou copolymères, on entend par polyamide les produits de condensation :
- d'un ou plusieurs aminoacides, tels les acides aminocaproïques, amino-7-heptanoïque, amino-11-undécanoïque et amino-12-dodécanoïque
- d'un ou plusieurs lactames tels que caprolactame, oenantholactame, le capryllactame et lauryllactame ;
- d'un ou plusieurs sels ou mélanges de diamines telles l'hexaméthylènediamine, la dodécaméthylènediamine, la méthaxylyènediamine, le bis-p aminocyclohexylméthane et la triméthylhexaméthylène diamine avec des diacides tels que les acides isophtalique, téréphtalique, adipique, azélaïque, subérique, sébacique et dodécanedicarboxylique.

On entend aussi les poudres fines obtenues à partir d'un monomère qui est un lactame que l'on transforme directement en poudre fine de polyamide.

A titre d'exemple de lactames, on peut citer ceux ayant de 3 à 12 atomes de carbone sur le cycle principal et pouvant être substitués. On peut citer par exemple le β, β-diméthylpropriolactame, le α,α-diméthylpropriolactame, l'amylolactame, le caprolactame, le capryllactame et le lauryllactame.

La méthode consiste à mettre le lactame en suspension dans un liquide organique ou en solution dans un solvant et d'effectuer une polymérisation de type anionique permettant d'obtenir directement de la poudre de PA qui se sépare d'elle-même du milieu liquide au fur et à mesure de sa formation. La méthode de polymérisation anionique des lactames est fondée essentiellement sur l'utilisation d'un catalyseur tel que le sodium ou un de ses composés comme l'hydrure de sodium ou le méthylate de sodium et d'un activateur tel que les lactame-N-carboxy-anilides, les isocyanates, les carbodi-imides, les cyanimides, les acyl-lactomes, les triazines, les urées, les imides-N-substituées et les esters entre autres en présence éventuellement d'une charge minérale ou organique finement divisée ayant un rôle de germe de cristallisation tel que de la poudre de PA, de la silice, du talc et en présence d'une N,N'-alkylène bis amide plus particulièrement la N, N'éthylène bis stéaramide, la N,N'éthylène bis oléamide, la N, N'éthylène bis palmitamide, gadoléamide, cétoléamide et érucamide, la N, N'-dioléyldipamide et la N, N'diérucylamide. Le procédé est décrit dans les brevets EP192515 et EP303530.

A titre d'exemple de polyamides utilisables dans l'invention, on peut citer le PA 6, le PA 6-6, le PA 11 et le PA 12.

Comme poudres de PA, on peut citer :
■ les poudres de Polyamides 12 non sphériques vendues par différentes sociétés : Nylon 2159V et Nylon 2070V (Société KOBO), Covabead N12 et Covabead N12-10 (Société LCW), Vestosint 7010 BC, Vestosint 7020 BC et Vestosint 7040 BC (Société Degussa)
■ les poudres de polyamides 12 sphériques vendues par différentes sociétés : SP-500, SP501, SP10 (Société TORAY), UBESTA (Société UBE), GPA 700 (Société Ganz Chemical)
■ les poudres sphéroïdales poreuses commercialisées par ARKEMA sous la dénomination Orgasol® : Orgasol® 2002 UD NAT COS, Orgasol® 2002 EXD NAT COS, Orgasol® 2002 EXD NAT COS Type S, Orgasol® 2002 D NAT COS, Orgasol® 1002 D NAT COS et Orgasol® 1002 EXD BL10 COS et sous la dénomination Orgasol® imprégnés : Orgasol® 1002 BL10PX COS imprégné à 20% en octylméthoxycinnamate, Orgasol® 2002 N5PX COS imprégné à 20% en octylméthoxycinnamate, Orgasol® 2002 N5HY COS imprégné à 40% d'une solution d'acide hyaluronique, Orgasol®N10S COS imprégné à 5% de diméthicone, Orgasol® 2002 N10VE COS imprégné à 12% d'acétate de tocophéryl et Orgasol® 2002 N10VB COS imprégné à 10% de D-Panthénol.

S'agissant des copolyamides, on peut citer les copolyamides résultant de la condensation d'au moins deux acides alpha oméga aminocarboxyliques ou de deux lactames ou d'un lactame et d'un acide alpha oméga aminocarboxylique. On peut encore citer les copolyamides résultant de la condensation d'au moins un acide alpha oméga aminocarboxylique (ou un lactame), au moins une diamine et au moins un acide dicarboxylique. On peut encore citer les copolyamides résultant de la condensation d'une diamine aliphatique avec un diacide carboxylique aliphatique et au moins un autre monomère choisi parmi les diamines aliphatiques différentes de la précédente et les diacides aliphatiques différents du précédent.

Les lactames que l'on peut utiliser sont les mêmes que ceux cités précédemment.

A titre d'exemple d'acide alpha oméga aminocarboxylique, on peut citer l'acide amino-undécanoïque et l'acide aminododécanoïque.

A titre d'exemple d'acide dicarboxylique, on peut citer l'acide adipique, l'acide sébacique, l'acide isophtalique, l'acide butanedioïque, l'acide 1,4 cyclohexyldicarboxylique, l'acide téréphtalique, le sel de sodium ou de lithium de l'acide sulphoisophtalique, les acides gras dimérisés(ces acides gras dimérisés ont une teneur en dimère d'au moins 98% et sont de préférence hydrogénés) et l'acide dodécanédioïque HOOC-(CH₂)₁₀-COOH.

A titre d'exemple de diamine, on peut être une diamine aliphatique ayant de 6 à 12 atomes, elle peut être arylique et/ou cyclique saturée. A titre d'exemples on peut citer l'hexaméthylènediamine, la pipérazine, la tetraméthylène diamine, l'octaméthylène diamine, la décaméthylène diamine, la dodécaméthylène diamine, le 1,5 diaminohexane, le 2,2,4-triméthyl-1,6-diamino-hexane, les polyols diamine, l'isophorone diamine (IPD), le méthyl pentaméthylènediamine (MPDM), la bis(aminocyclohéxyl) méthane (BACM), la bis(3-méthyl-4 aminocyclohéxyl) méthane (BMACM).

A titre d'exemples de copolyamides, on peut citer des copolymères de caprolactame et de lauryle lactame (PA 6/12), des copolymères de caprolactame, d'acide adipique et d'hexaméthylène diamine (PA 6/6-6), des copolymères de caprolactame, de lauryle lactame, d'acide adipique et d'hexaméthylène diamine (PA 6/12/6-6), des copolymères de caprolactame, de lauryle lactame, d'acide amino 11 undécanoïque, d'acide azélaïque et d'hexaméthylène diamine (PA 6/6-9/11/12), des copolymères de caprolactame, de lauryle lactame, d'acide amino 11 undécanoïque, d'acide adipique et d'hexaméthylène diamine (PA 6/6-6/11/12), des copolymères de lauryle lactame, d'acide azélaïque et d'hexaméthylène diamine (PA 6-9/12).

On peut utiliser des mélanges de polyamide et/ou de copolyamide. Ce sont par exemple des mélanges de polyamides aliphatiques et de polyamides semi-aromatiques et des mélanges de polyamides aliphatiques et de polyamides cycloaliphatiques.

Les poudres peuvent être fabriquées par tout moyen, dissolution et précipitation dans un alcool. Avantageusement, on produit par polymérisation dans un solvant, les poudres étant insolubles dans un ce solvant (polymérisation de type anionique définie plus haut). On peut citer le procédé décrit dans EP192515 et EP303530.

On peut aussi utiliser des poudres de copolyesteramides comprenant en moles (le total étant 100%) :
■ 1 à 98% d'un lactame,
■ 1 à 98% d'une lactone, et éventuellement
■ 1 à 98% d'un autre lactame,
et qui ont un diamètre compris entre 1 µm et 200 µm, une surface spécifique comprise entre 1 et 25 m²/g.

Les lactames que l'on peut utiliser pour fabriquer les copolyesteramides sont les mêmes que ceux cités plus haut. On utilise avantageusement le caprolactame et le lauryllactame.

A titre d'exemple de lactones, on peut citer la caprolactone, la valérolactone et la butyrolactone. On utilise avantageusement la caprolactone.

Le procédé de préparation de ces poudres de copolyesteramides par polymérisation anionique est décrit dans le document EP1172396.

On peut aussi utiliser des poudres de polyuréthanes, de polymères acryliques, de polyesters, de silicones, de polyéthylènes, de silices.
Comme poudre de silice, on peut citer :
- les microsphères de silice poreuses vendues sous la dénomination SILICA BEADS SB-700 par la société MYOSHI ; "SUNSPHERE® H51", "SUNSPHERE® H33" par la société ASAHI GLASS ;
- les microsphères de silice amorphe enrobées de polydiméthylsiloxane vendues sous la dénomination "SA SUNSPHERE® H 33", "SA SUNSPHERE® H53" par la société ASAHI GLASS.
Comme poudre de polymères acryliques, on peut citer :
- les poudres de polyméthacrylate de méthyle vendus sous la dénomination COVABEAD® LH85 par la société LCW;
- les poudres de poly méthacrylate de méthyle/diméthacrylate d'éthylène glycol vendues sous la dénomination DOW CORNING 5640 MICROSPONGE® SKIN OIL ADSORBER par la société DOW CORNING ; GANZPEARL® GMP-0820 par la société GANZ CHEMICAL;
- les poudres de polyméthacrylate d'allyle/diméthacrylate d'éthylène glycol vendues sous la dénomination POLY-PORE® L200, POLY- PORE® E200 par la société AMCOL ;
- les poudres de copolymère diméthacrylate d'éthylène glycol/méthacrylate de lauryle vendues sous la dénomination POLYTRAP® 6603 de la société DOW CORNING.

Comme poudre de silicone élastomère, on peut citer les poudres vendues sous les dénominations "Trefil® Powder E-505C", "Trefil® Powder E-506C" par la société DOW CORNING.
Avantageusement, les particules de poudres ont une forme sphéroïdale.

### PHASE GRASSE

Une phase grasse peut contenir une phase grasse liquide et éventuellement une phase grasse solide (telles que les cires). La phase grasse liquide peut contenir une ou plusieurs huiles liquides à température ambiante (25°C), ces huiles sont volatiles ou non. La phase grasse liquide est formée d'huiles hydrocarbonées voir éventuellement d'huiles de silicones.

La phase grasse de la composition est une phase grasse continue qui avec l'eau fournie une émulsion sous la forme eau dans huile. Cette phase grasse comprend une ou plusieurs huiles, c'est à dire des corps gras non miscibles à l'eau. Ces huiles volatiles ou non, sont d'origine minérale, animale, végétale ou de synthèse et peuvent être hydrocarbonées, siliconées ou fluorées. Par huile hydrocarbonée, on entend une huile formée essentiellement, voire constituée, d'atomes de carbone et d'hydrogène et éventuellement d'atome d'oxygène, d'azote. Elle peut contenir des groupes alcool, ester, éther, acide carboxylique, amine et/ou amide.

Elle peut contenir une ou plusieurs huiles liquides à température ambiante (25°C), de préférence au moins une huile liquide non volatile. On entend par huile liquide non volatile, une huile susceptible de rester sur la peau à température ambiante (25°C) et pression atmosphérique au moins une heure et ayant notamment une pression de vapeur à température ambiante (25°C) et pression atmosphérique, non nulle, inférieure ou égale à 0,01 mm de Hg (1,33 Pa).

La phase grasse liquide comprend avantageuement une ou plusieurs huiles non volatiles qui procurent un effet émollient sur la peau. On peut citer les esters gras tels que l'isononoate de cetearyl, l'isononoate d'isotridecyl,l' isostearate d'isostearyl, l'isostearate d'isopropyl, le myristate d'isopropyl, le palmitate d'isopropyl, stéarate de butyle, le laurate d'hexyle, l'isononate d'isononyle, le palmitate de 2-éthyl-hexyle, le laurate de 2-hexyldécyle, le palmitate de 2-octyl décyle, le myristate ou la lactate de 2-octyldodécyle, le succinate de 2-diéthyl hexyle, le malate de diisostéaryle, le triisostéarate de glycérine ou de triglycérine, l'acetate de tocopherol, les acides gras supérieurs tels que l'acide myristique, l'acide palmitique, l'acide stéarique, l'acide béhénique, l'acide oléique, l'acide linoléique, l'acide linolénique ou l'acide isostéarique, l'acide caprylique/caprique triglyceride, les alcools gras supérieurs tel que l'alcool oléique, l'huile d'avocat, l'huile de camélias, l'huile de noix de macadamia, l'huile de tortue, l'huile de vison, l'huile de soja, l'huile de pépins de raisin, l'huile de sésame, l'huile de maïs, l'huile de colza, l'huile de tournesol, l'huile de coton, l'huile de jojoba, l'huile d'arachide, l'huile d'olive, l'hexyl laurate et leurs mélanges.

Elles peuvent être des huiles minérales : les huiles d'hydrocarbures telles que l'huile de paraffine, de squalane, la vaseline et leurs mélanges.

Eventuellement la composition comprend des huiles de silicone non volatiles comme par exemple les diméthyls siloxanes.

La phase grasse liquide peut aussi comprendre éventuellement des huiles volatiles. Par huile volatile, on entend une huile susceptible de s'évaporer de la peau, en moins d'une heure à température ambiante et pression atmosphérique. Cette huile a notamment une pression de vapeur, à température ambiante (25°C) et pression atmosphérique (760 mm Hg) supérieure à 0,01 et inférieur ou égale à 300 mm de Hg (1,33 Pa à 40 000 Pa) et de préférence allant de 0,05 à 300 mm de Hg (6,65 Pa à 40 000 Pa).

Les huiles volatiles sont par exemple choisies parmi les huiles de silicones qui contribuent à réduire l'effet gras des formulations à phase grasse continue. On peut citer les huiles de silicones linéaires ou cycliques ayant une viscosité à température ambiante inférieure à 8 mm²/s et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'actaméthyl cyclotétrasiloxane, le décaméthyl cyclopentasiloxane, le dodécaméthyl cyclohexasiloxane, l'heptaméthyl hexyl trisiloxane, l'heptaméthyl octyl trisiloxane, l'hexaméthyl disiloxane, l'octaméthyl trisiloxane, le décaméthyl tétrasiloxane, le dodécaméthyl pentasiloxane et leurs mélanges.

Elles sont plus particulièrement de la famille des polyalkyl ou polyaryl siloxanes : le cyclométhicone (DC 345 de Dow Corning), le caprylyl méthicone, le cyclopentasiloxane (DC245 de Dow Corning).

On peut aussi citer les huiles volatiles hydrocarbonées ayant de 8 à 16 atomes de carbone et leurs mélanges et notamment les alcanes ramifiés en C₈ à C₁₆ comme les isoalcanes (appelés aussi les isoparaffines) en C₈ à C₁₆, l'isododécane, l'isodécane, l'isohexadécane, les esters ramifiés en C₈ à C₁₆ comme le néo pentanoate d'isohexyle et leurs mélanges.

Avantageusement, la composition selon l'invention contient au plus 25% d'huile volatile et notamment d'huile de silicone volatile, de préférence au plus 15% (% en poids par rapport à la composition totale).

### PHASE AQUEUSE

La phase aqueuse contient de l'eau. Cette dernière peut être une eau florale telle que l'eau de bleuet et/ou une eau minérale telle que l'eau de VITTEL, l'eau de LUCAS, l'eau de LA ROCHE POSAY et/ou une eau thermale. La phase aqueuse peut également comprendre des constituants miscibles à l'eau comme par exemple les alcools primaires tels que l'éthanol et l'isopropanol, les polyols tels que les glycols ajoutés pour leurs propriétés humectantes: le glycérol, le propylène glycol, le butylène glycol, le dipropylène glycol, le diéthylène glycol, les éthers de glycol tel que les alkyl(C1-C4)éther de mono, di ou tripropylène glycol, mono, di ou triéthylène glycol et leurs mélanges.

La phase aqueuse peut comprendre en outre des agents de stabilisation tels que le chlorure de sodium, le dichlorure de magnésium et le sulfate de magnésium.

La phase aqueuse peut également comprendre tous composés hydrosolubles ou hydrodispersibles compatibles avec une phase aqueuse tels que les gélifiants, les polymères filmogènes, les épaississants, les tensio-actifs et leur mélange.

### AUTRES COMPOSES

La composition cosmétique selon l'invention peut également comprendre des tensio-actifs ( généralement lipophiles) de type anionique, non ionique ou amphotère facilitant la dispersion de la phase aqueuse dans la phase grasse de façon à obtenir une émulsion eau/huile stable, des additifs tels que des conservateurs (généralement hydrophiles), des parfums (généralement lipophiles), des charges différentes de la poudre selon l'invention, des matières colorantes (colorants solubles, pigments), des épaississants (cires, gélifiants), des stabilisants d'émulsion (généralement hydrophiles), des chélateurs (généralement hydrophiles).

Les tensio-actifs peuvent être de type ester tels que les dérivés du sorbitan (ex sorbitan sesquiisostearate), le methyl glucose isostearate. Ils peuvent être de types polymériques tels que le PEG-45/dodécylglycol copolymère. Ils peuvent également être des tensioactifs silicones adaptés à la mise en émulsion des huiles de silicones : il s'agit par exemple de diméthicone copolyols comme le PEG/PPG-18/18 diméthicone commercialisé par Dow Corning sous le nom DC5225C.

Les épaississants peuvent être par exemple solubles dans la phase grasse pour ajuster sa consistance ou contribuer à la stabilité de la composition : on peut citer par exemple la cire de candellila, des gommes ou des élastomères de silicones (DC1411 et DC9040 de Dow Corning).

Les conservateurs sont des mélanges de dérivées des paraben et/ou de phenoxyethanol.

On peut citer par exemple l'EthylèneDiamineTétraAcétique (EDTA) comme agent chélatant.

Elle peut aussi comprendre des actifs cosmétiques qui procurent une amélioration des matières kératiniques humaines cités plus haut. Les actifs cosmétiques comprennent les hydratants (généralement hydrophiles) tels que les polyols, les bloqueurs de rayonnements UV tels que les filtres organiques (généralement lipophiles) ou les particules minérales telles que TiO2, ZnO traitées en surface ou non, les anti-rides (généralement hydrophiles), les autobronzants (généralement hydrophiles), les filmogènes (lipophiles ou hydrophiles selon leur nature), les antioxydants (lipophiles ou hydrophiles selon leur nature).

On peut citer comme filtres minéraux des dispersions de ZnO et de Ti02 dans des mélanges d'huiles de silicones.

La composition cosmétique selon l'invention comprend avantageusement:
- 10 à 75%, de préférence de 30 à 65% de phase aqueuse ;
- 0,1 à 30%, de préférence de 1 à 20% de poudre selon l'invention ; et
- 89,9 à 24,9% de phase grasse, le total faisant 100% (% en poids).

La phase aqueuse comprend pour sa part de préférence 10 à 60% de polyols par rapport à la phase aqueuse totale.

De plus elle peut comprendre 0,5 à 10%, de préférence 3 à 5% de tensioactifs, 0,01 à 2% d'additifs, 0,005 à 10% d'actifs cosmétiques par rapport à la composition totale.

Les caractéristiques des poudres de la composition, objet de l'invention sont :
1. la taille des particules très fine de 2 µm à 100 µm, de préférence de 2 à 50, encore plus avantageusement de 2 à 20,
2. la répartition granulométrique étroite.
   La distribution granulométrique des poudres est déterminée selon les techniques habituelles par exemple à l'aide d'un granulomètre Coulter Multisizer Il selon la norme ISO 13319. A partir de la distribution granulométrique, il est possible de déterminer le diamètre moyen ainsi que la dispersion granulométrique (écart-type) qui mesure le resserrement de la distribution. C'est l'un des avantages du procédé décrit que de permettre d'obtenir une distribution resserrée avec un écart-type compris entre 1 et 3 ∼m, voire souvent inférieur à 2 µm
3. la forme des particules de façon avantageuse sphéroïdale, c'est à dire en forme de sphéroïde qui signifie : solide à peu près sphérique.
4. la porosité de surface caractérisée par la surface spécifique apparente (SSA) des poudres de 0,5 à 299 m²/g, de préférence de 0,5 à 150 m²/g, encore plus préférentiellement de 0,5 à 100 m²/g, avantageusement de 0,5 à 50 m²/g, encore plus avantageusement de 0,5 à 40 m²/g, mesurée par absorption d'azote selon la méthode BET définie ci-dessous.
5. une Absorption d'huile de lin entre 45g/100g et 160g/100g, de préférence entre 50 g/100g et 150 g/100g mesurée selon la méthode définie plus bas.

Les caractéristiques énumérées ci-dessus contribuent fortement au toucher doux et à l'obtention après application de l'aspect mat et poudré de la composition, interviennent dans l'absorption d'une partie de la phase grasse et contribuent à limiter les effets négatifs de ladite phase grasse (sensation de gras, de huileux, de poisseux).

La surface spécifique apparente SSA est déterminée selon la méthode BET (Brunauer-Emmet-Teller) décrite dans la norme internationale ISO 9277. La surface spécifique BET correspond à la surface spécifique totale (donc micropores compris) de la poudre.

La méthode de prise de l'huile de lin ou absorption de l'huile de lin d'une poudre est décrite dans la norme ISO 787-5. Elle correspond à la quantité d'huile adsorbée sur la surface disponible de la poudre. On place une quantité m (en grammes) de poudre comprise entre environ 0,5 g et 5 g dans un bécher en verre puis on ajoute goutte à goutte de l'huile de lin. Après addition de 4 à 5 gouttes, on incorpore l'huile dans la poudre à l'aide d'une spatule et on continue d'ajouter l'huile jusqu'à formation de conglomérats d'huile et de poudre. A partir de ce moment, on ajoute l'huile à raison d'une goutte à la fois et on triture ensuite le mélange avec une spatule. On cesse l'addition de l'huile lorsque l'on obtient une boule solide. On note alors la masse m'(exprimée en g) de l'huile utilisée. La prise d'huile de lin ou absorption d'huile de lin est exprimée en m/m' ( g d'huile/ g de poudre) est ensuite convertie en g/100g de poudre.

Les exemples des **TABLEAUX 1 à 4** ci-dessous sont définis de la façon suivante :
1. **Poudre 1 : Orgasol®2002EXD NAT COS** c'est à dire poudre de PA12, taille des particules 10 µm, SSA 4 +/-1,5 m²/g, prise d'huile de lin de 79g/100g.
2. **Poudre 2: Orgasol®2002D NAT COS** poudre de PA12, taille des particules 20 µm, SSA 1,5 +/-1 m²/g, prise d'huile de lin de 54g/100g.
3. **Poudre 3: Orgasol®1002D NAT COS** poudre de PA6, taille des particules 20 µm, SSA 2,5 +/-1 m²/g, prise d'huile de lin de 64g/100g.
4. La composition **TEMOIN** ne comprend pas de poudre.
Les pourcentages ci-dessous sont exprimés en poids par rapport à la composition totale.
La nature des compositions comprenant les Poudres 1 à 4 est définie plus bas pour chaque tableau.

L'effet de l'addition de poudres PA selon l'invention dans des émulsions à phase grasse continue a été mesuré par analyse sensorielle dans différents types de compositions. Chaque composition a fait l'objet d'une étude de profil sensoriel, conduite par un panel de cinq experts selon les descriptifs suivants :
- pendant la phase d'application du produit : le gras, le huileux, la rapidité de pénétration
- immédiatement après application : le brillant de la peau, la douceur de la peau, l'effet peau grasse, l'effet peau collante, le résidu laissé par la crème sur la peau.

Chaque composition est analysée en aveugle par comparaison de tous les essais formant une série.

Les résultats sont rassemblés dans les **TABLEAUX 1 à 4**. Les différents critères ont été évalués sur une échelle allant de 0 à 8. La valeur 0 indiquant l'absence du critère désigné (par exemple une sensation d'absence de gras) ; la valeur 8 indiquant une tendance très marquée pour le critère choisi (par exemple une sensation très importante de présence de gras).

➢ Les **Compositions A à C** et le **Témoin 1** du **TABLEAU 1** sont des compositions de type émulsions Eau dans Silicones, comprenant de forte teneur en glycérine correspondant par exemple à des compositions de crèmes de jours.

Le procédé de préparation des compositions ci-dessous consiste à (i) combiner la phase aqueuse, (ii)combiner la phase grasse, (iii) ajouter la phase aqueuse à la phase grasse lentement tout en agitant énergiquement de façon à former une émulsion puis (iv) à ajouter lentement la poudre (hormis pour le témoin) en agitant doucement.

| **Témoin 1 et Compositions A à C** | | |
|---|---|---|
| **milieu** | **%** | **Ingrédients / Fournisseur** |
| **Phase aqueuse** | QSP 100 | Eau déminéralisée |
| | 27.0 | Glycérine |
| | 13.5 | Butylène glycol |
| | 1.0 | NaCl |
| | | |
| **Phase grasse** | 9.0 | DC 5225 C (1) (cyclopentasiloxane et PEG/PPG-18/18 diméthicone) |
| | 1.0 | DC 9040 (1) (cyclométhicone et diméthicone copolymères) |
| | 8.0 | DC 345 (1) (cyclométhicone) |
| | 3.5 | DC1411 (1) (cyclométhicone et diméthicone) |
| | 2 | Silcare 41 M15 (2) (caprylyl méthicone) |
| | | |
| **Poudre** | x | Poudre 1 à 3 selon composition A à C |

| | | |
|---|---|---|
| (1) Dow Corning; (2) Clariant **Témoin 1 :** x= 0% de poudre. **Composition A :** x = 3,5% de poudre 1. **Composition B :** x = 3,5% de poudre 2. **Composition C :** x = 3,5% de poudre 3. | | |

**TABLEAU 1**

| | | **Témoin 1** | **Composition A** | **Composition B** | **Composition C** |
|---|---|---|---|---|---|
| Comportement lors de lors de l'application | Gras | 8 | 3 | 2 | 5 |
| | Huileux | 8 | 3 | 2 | 5 |
| | Rapidité de pénétration | 0 | 4 | 5,5 | 2 |
| Comportement après l'application | Brillant | 8 | 4 | 2 | 5 |
| | Douceur | 2 | 4 | 7 | 5 |
| | Peau grasse | 8 | 6 | 2 | 5 |
| | Peau collante | 7 | 5 | 1 | 5 |
| | Résidu | 8 | 5 | 1 | 5 |

➢ Les **Compositions D à G** et le **Témoin 2** du **TABLEAU 2** sont des compositions de type émulsions Eau dans Silicones, pouvant correspondre par exemple à des fluides hydratants doux.
Le procédé de préparation des compositions ci-dessous consiste à (i) combiner les conservateurs à 60°C (nommée phase aqueuse 2) et à les ajouter à la phase aqueuse (nommée phase aqueuse 1), à (ii)combiner la phase grasse tout en agitant avec un équipement Rayneri dans un bain froid, à (iii) ajouter lentement sous agitation le mélange phase aqueuse 1 + phase aqueuse 2 préparé en (i) à la phase grasse préparée en (ii) puis à (iv) ajouter lentement la poudre (hormis pour le témoin) en agitant doucement.

| **Témoin 2 et Compositions de D à G** | | |
|---|---|---|
| **milieu** | **%** | **Ingrédients / Fournisseur** |
| **Phase aqueuse 1** | QSP 100 | Eau déminéralisée |
| | 2.0 | NaCl |
| | | |
| **Phase aqueuse 2** | 0.6 | Phénonip (phénoxyéthanol et méthylparaben et éthylparaben et buthylparaben et propylparaben et isobutylparaben) (1) |
| | 0.2 | Chlorphénésin (2) |
| | 8.0 | Glycérine |
| **Phase grasse** | 10.0 | DC 5225C (cyclopentasiloxane et PEG/PPG-18/18 diméthicone) (3) |
| | 10.0 | DC 345 (cyclométhycone) (3) |
| | 10.0 | DC 245 ( cyclométhicone ou cyclopentasiloxane) (3) |
| | | |
| **Poudre** | x | Poudre 1 à 3 selon compositions D à G |

| | | |
|---|---|---|
| (1) Clariant ; (2) Arnaud ; (3) Dow Corning **Témoin 2 :** x = 0% de poudre. **Composition D :** x = 3,5% de poudre 1. **Composition E :** x = 10% de poudre 1. **Composition F:** x = 3,5% de poudre 2. **Composition G:** x = 3,5% poudre 3. | | |

**TABLEAU 2**

| | | **Témoin 2** | **Composition D** | **Composition E** | **Composition F** | **Composition G** |
|---|---|---|---|---|---|---|
| Comportement lors de l'application | Huileux | 7 | 2,5 | 0 | 7 | 3 |
| | Rapidité de pénétration | 1 | 5 | 7 | 1 | 5 |
| Comportement après l'application | Brillant | 4 | 1 | 0 | 4 | 1 |
| | Douceur | 2 | 7 | 8 | 4 | 6 |

L'addition de 1 à 30% en poids, de préférence de 2 à 10%, de poudres de PA dans une émulsion Eau dans Silicones (type crème de jour, fluide hydratant, lait corporel, soin après-rasage entre autre) permet de réduire significativement la sensation de gras et de poisseux après application sur la peau. Par ailleurs, après application, l'aspect brillant et huileux de la peau est complètement supprimé.

L'addition de poudre permet même au contraire d'obtenir un aspect mat et poudré, et confère à la peau un toucher doux.

En particulier, dans une composition du type témoin 1 contenant une forte teneur en polyol (plus de 50% de la phase aqueuse) et une teneur élevée en composés siliconés non volatile (plus de 50% de la phase grasse), l'addition de poudre diminue la sensation de gras et le collant résiduel sur la peau.

En particulier, dans une composition du type témoin 2 contenant des polyols et des silicones volatiles, l'addition de poudre PA permet de supprimer l'effet gras associé aux huiles de silicones et aux polyols pendant l'application. Cela permet aussi, après application, d'obtenir un fini poudré et une sensation douce malgré l'évaporation des huiles de silicones volatiles alors qu'en absence de la poudre selon l'invention, on obtient un effet désagréable.

➢ Les **Compositions H à J** et le **Témoin 3** du **TABLEAU 3** sont des compositions comprenant de type émulsions Eau dans Huile. Ces compositions correspondent par exemple aux formulations de crèmes solaires « waterproof ».

Le procédé de fabrication de la formulation ci-dessous consiste (1) à mélanger les constituants de la phase aqueuse en chauffant à 70°C, (2) à mélanger les constituants de la phase grasse (nommée phase grasse 1) en chauffant à 70°C. L'épaississant (nommé phase grasse 2), et les filtres solaires sont ensuite ajouter à la phase grasse maintenue à 70°C. Enfin, la phase aqueuse est ajoutée dans la phase grasse sous agitation cisaillante afin d'obtenir l'émulsion. L'émulsion est ensuite refroidie à température ambiante et la poudre de Polyamide est dispersée dans l'émulsion sous agitation douce.

| **Témoin 3 et Compositions H à J** | | |
|---|---|---|
| **Milieu** | **%** | **Ingrédients / Fournisseur** |
| **Phase aqueuse** | QSP 100 | Eau déminéralisée |
| | 0.70 | Sulfate de magnésium (1) |
| | 0.10 | EDETA BD (disodium EDTA) (2) |
| | 5.00 | Glycerine |
| | | |
| **Phase grasse 1** | 3.50 | Isolan IS (méthyl glucose isostearat) (3) |
| | 1.00 | Elfacos ST9 (PEG-45/dodécylglycol copolymer) (3) |
| | 11.00 | Cétiol A (hexyl laurate) (4) |
| | 5.00 | Cétiol SN (cetearyl isononanoate) (4) |
| | 10.00 | Cétiol S (diéthylhexylcyclohexane) (4) |
| | | Abil SOFT AF100 (methoxy PEG/PPG-7/3/aminopropyl |
| | 3.00 | diméthicone) (3) |
| | 0.50 | Candellila wax (4) |
| | 0.80 | Phénonip (phénoxyéthanol et méthylparaben et éthylparaben et |
| | | buthylparaben et propylparaben et isobutylparaben) (5) |
| | 0,28 | Chlorphenesin (6) |
| | 0.20 | Butylparaben (5) |
| | 0.15 | Dehydroacetic Acid (7) |
| | 1.00 | PVP/Eicosene Copolymer (Antaron V-220) (8) |
| | | |
| **Phase grasse 2** | 1.50 | Disteardimonium hecto rite (Bentone 38VCG) (4) |
| | | |
| **Filtres minéraux Poudre** | 2.50 | Zinc Oxide + Triethoxycaprylylsilane (Z Cote HP1) (2) |
| | | |
| | 14.00 | Titanium dioxide (and) Isononyl Isonanoate (and) Stearic Acid (and) Aluminium hydroxide (KOBO IN60S4) (9) |
| | | |
| **Poudre** | x | Poudre 1 à 3 selon compositions H à J |

| | | |
|---|---|---|
| (1) Merck, (2)Laserson, (3)Goldschmidt, (4)Saci, (5)Clariant, (6)Arnaud, (7)Sigma, (8)ISP, (9)KOBO **Témoin 3 :** x = 0% de poudre. **Composition H :** x = 3,5% poudre 1. **Composition I :** x = 3,5% poudre 2. **Composition J:** x = 3,5% poudre 3. | | |

**TABLEAU 3**

| | | **Témoin 3** | **Composition H** | **Composition I** | **Composition J** |
|---|---|---|---|---|---|
| Comportement lors de l'application | Gras | 7 | 6 | 4 | 3 |
| | Huileux | 7 | 6 | 4 | 3 |
| | Rapidité de pénétration | 0 | 1 | 2 | 3 |
| Comportement après l'application | Brillant | 7 | 6 | 5 | 4 |
| | Douceur | 2 | 4 | 5 | 6 |
| | Peau grasse | 7 | 6 | 5 | 4 |
| | Peau collante | 2 | 2 | 0 | 0 |
| | Résidu | 7 | 7 | 3 | 2 |

De même, l'addition de 1 à 30% en poids, de préférence de 2 à 10%, de poudres de PA dans une émulsion Eau dans Huile (type crème solaire waterproof) permet de réduire significativement la sensation de gras et de poisseux après application sur la peau. Par ailleurs, après application, l'aspect blanc de la peau dû aux filtres solaires physiques (Oxyde de Zinc, dioxyde de titane), est fortement atténué.

➢ Les **Compositions D et E** et le **Témoin 2** sont décrits plus haut.

**TABLEAU 4**

| | **Témoin 2** | **Compositions D** | **Composition E** | **Poudre Polyamide non sphéroïdale*** 3,5% |
|---|---|---|---|---|
| Douceur après l'application | 2 | 7 | 4 | 2 |

| | | | | |
|---|---|---|---|---|
| * Poudre PA à particules non sphéroïdales, constituée de grains anguleux et de tailles variables, taille moyenne 8 µm et de SSA 4 m²/g. | | | | |

Avantageusement, on observe une amélioration de la douceur de la peau après application de la crème sur celle-ci grâce à la présence de la poudre fine sphéroïdale dans la composition selon l'invention.

## Revendications

**1.** Emulsion cosmétique eau/huile comprenant comme seule poudre, une poudre fine et poreuse de polyamide, les particules de poudre présentant les caractéristiques suivantes :
- une forme sphéroïdale ;
- une surface spécifique apparente de 0,5 à 40 m²/g mesurée par absorption d'azote selon la méthode BET ISO 9277;
- une absorption d'huile de lin entre 45g/100g de poudre et 160g/100g de poudre mesurée selon ISO 787-5.

**2.** Emulsion selon la revendication 1, **caractérisée en ce que** la poudre présente une absorption d'huile de lin de 50 g/100g de poudre à 150 g/100g de poudre.

**3.** Emulsion selon l'une des revendications précédentes, **caractérisée en ce que** les particules de poudre ont un diamètre moyen allant de 2 µm à 100 µm, de préférence de 2 à 50 µm, encore plus avantageusement de 2 à 20 µm.

**4.** Emulsion selon l'une des revendications précédentes, dont la composition est la suivante :
• 10 à 75%, de préférence 30 à 65% d'une phase aqueuse ;
• 0,1 à 30%, de référence 1 à 20% de poudre poreuse; et
• 89,9 à 24,9% d'une phase grasse, le total faisant 100%.

**5.** Emulsion selon la revendication 4, **caractérisée en ce que** la phase grasse comprend moins de 25% (en poids par rapport à la composition totale) d'huile volatile.

**5.** Emulsion selon la revendication 4, **caractérisée en ce que** l'huile volatile est une huile siliconée.

**7.** Emulsion selon l'une des revendications 4 à 6, **caractérisée en ce que** la phase aqueuse comprend de 10 à 60% de polyols.

**8.** Emulsion selon l'une des revendications précédentes, **caractérisée en ce que** l'émulsion comprend un ingrédient cosmétique choisi parmi les antioxydants, les parfums, les conservateurs, les neutralisants, les tensioactifs, les polymères filmogènes, les épaississants, les bloqueurs de rayonnements ultra violet, les vitamines, les matières colorantes, les stabilisants d'émulsion, les hydratants, les composés auto-bronzants, les actifs antirides et leurs mélanges.

**9.** Emulsion selon l'une des revendications précédentes, **caractérisée en ce que** l'émulsion est une crème ou un fluide de soin du visage, une crème ou un fluide de soin du corps hydratant et/ou amincissant, une crème solaire résistante à l'eau ou non résistante à l'eau, un fond de teint, un fard à paupières, un fard à joue, un produit anti-cernes ou un produit de maquillage du corps.

**10.** Utilisation d'une émulsion selon l'une des revendications précédentes pour fabriquer un maquillage et/ou de soin de la peau non brillant, non huileux, non poisseux et/ou non collant.

**11.** Procédé cosmétique de maquillage et/ou de soin des matières kératiniques, comprenant l'application sur ces matières d'une émulsion obtenue par une utilisation selon l'une quelconque des revendications 1 à 10.

**12.** Emulsion selon l'une quelconque des revendications 1 à 9, dans laquelle ledit polyamide est choisi parmi le PA 6, PA 6-6, PA 11 et le PA12.
